(19) Europäisches Patentamt
European Patent Office
Office européen des brevets

(11) **EP 4 427 672 A1**

(12) **EUROPEAN PATENT APPLICATION**

(43) Date of publication:
**11.09.2024 Bulletin 2024/37**

(21) Application number: **23161315.9**

(22) Date of filing: **10.03.2023**

(51) International Patent Classification (IPC):
***A61B 5/08*** *(2006.01)* ***A61B 5/00*** *(2006.01)*

(52) Cooperative Patent Classification (CPC):
**A61B 5/0816; A61B 5/7235**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Lynx Health Science GmbH
57439 Attendorn (DE)**

(72) Inventor: **Klum, Michael
Berlin 13581 (DE)**

(74) Representative: **Gulde & Partner
Patent- und Rechtsanwaltskanzlei mbB
Wallstraße 58/59
10179 Berlin (DE)**

(54) **METHOD, DEVICE AND SYSTEM FOR DETECTING DISTINCT REPETITIVE EVENTS FROM SIGNALS**

(57) The present disclosure provides a method, device and system for detecting at least two distinct repetitive events (300), especially peaks and troughs, from a signal (100), by comprising a candidate detection (201) that selects respective candidates (210) for the distinct repetitive events (300) from the signal (100); a feature generation (202) in which candidate features (240) are captured; a state estimation (203) that adjusts at least one internal state (230) based on at least one candidate feature (220); and a candidate classification (204) that determines the distinct repetitive events (300) from the respective candidates (210) based on at least one candidate feature (220) and at least one internal state (230).

Fig. 1

EP 4 427 672 A1

## Description

### Field of the Invention

The invitation relates to the field of determining events in a signal and thus to pattern recognition.

### Technological Background

In a variety of signals there are several distinct repetitive events which represent meaningful values, such as respiratory peaks and troughs in a respiratory signal or electrical peaks in an electrocardiogram signal. For many of these events, automatic detection is necessary to save time and resources, this is especially true for events in biomedical signals.

However, automated detection of signal events is often not trivial because the signals are often noisy, complex, and highly variable. Many existing methods are either insufficiently powerful and robust in detection or have unreasonably high memory and computational requirements, which is particularly problematic on devices with limited resources, such as wearable devices.

Therefore, especially for biomedical signals, there is no adequate method for determining events on devices with limited resources. The cause of the deficiencies of the existing methods often lies in disadvantageous approaches for analyzing. First, there are methods that use fixed threshold decisions for detection, which produce inadequate results in terms of both robustness and performance but which are good in terms of limited memory and computational resources. Often, these methods attempt to compensate for the shortcomings in performance and robustness by using preprocessing steps (e.g., filtering), but in many cases this leads to consequential errors. On the other hand, there are very computationally intensive methods that use very complex feature generation or signal transformations, which is harmful to the implementability in systems with limited memory and computational capacity, but often lead to good performance without depending on preprocessing steps. Especially for biological live monitoring systems, which are often used with wearable devices, it is also crucial that all methods used can work with live data and are thus online-capable. Most existing methods lack this capability, often due to reliance on non-causal filters, non-causal feature generation, or non-causal feature transformation.

Therefore, a need exists for a method of detecting events in a signal that is powerful, has online functionality, and can be used on systems with limited memory and computational capacity. Such a method is disclosed in the present invention.

### General Description of the Invention

The present invention relates to a method that detects at least two distinct repetitive events from a signal by comprising a candidate detection that selects respective candidates for the distinct repetitive event from the signal; a feature generation in which candidate features are captured; a state estimation that adjusts at least one internal state based on at least one candidate feature; and a candidate classification that determines the distinct repetitive events from the respective candidates based on at least one candidate feature and at least one internal state.

As used herein, the term "signal" refers to a discretely sampled time signal, preferably stored digitally. According to some embodiments of the present invention, this signal is a biomedical signal or a respiratory signal, such as the flow signal of a respiratory mask or a derived respiratory signal from easily measurable signals.

As used herein, the term "event" refers to the point in time of an underlying activity or process evident in the present signal, especially a peak or trough. Thus, the distinct repetitive events are instances of a repetitive cyclic/rhythmic process or activity, such as a respiratory or a cardiac cycle or part thereof. The terms cyclic or rhythmic refer to the repeated nature or patterned nature of the event and/or to a sequential property thereof, for example, an expected sequence of phases in a cardiac cycle, an expected sequence of a respiratory cycle, an expected sequence of a circadian cycle, or the like, that commonly form a pattern associated with the activity. Such a repeated event can be involuntary, for example, a cardiac cycle or an involuntary respiratory cycle, or at least partially voluntary, such as guided respiration activity.

As used herein, the term "candidate" refers to a point in time in the present signal that indicates a possible event. A candidate may be a possible event of only one of the distinct repetitive events, or from several, or all of them. In any case, there must be candidates for each of the distinct repetitive events.

As used herein, the term "feature" refers to an individual measurable property or characteristic of the candidates. A feature may be a direct property of the signal (e.g., a maximum or minimum amplitude value) or it may be indirectly derivable from the signal (e.g., properties derived by applying analytical, statistical or model-based methods to the signal). Advantageously, the candidate features are informative, discriminative as to whether or not they are true events, and independent of each other.

As used herein, the term "internal state" refers to a variable that affects the behavior of the disclosed method but is not apparent from outside of the method. For example, a method according to the present invention may implement a parameter-based fitting algorithm for the step of candidate classification. The parameters for the fitting algorithms may be preset and defined as an internal state of a method according to the present invention. For state estimation, at least one internal state, i.e., at least one of the preset parameters, may be adjusted based on at least one candidate feature. Said candidate feature may thus have an actual effect on candidate classification. However, an internal state may also contain information about whether the event type of the last valid candidate was the expected event type. For example, an internal state may refer to simple Boolean information or a more complex data structure.

According to some embodiments of the present invention, the distinct repetitive events occur consecutively, for instance as in respiratory peaks and troughs, wherein another respiratory peak may occur only after the occurrence of a respiratory trough. Advantageously, this property is used in feature generation or state estimation, for example, by generating a feature or internal state that contains information about whether the event type of the last valid candidate was the expected event type.

According to some embodiments of the present invention, the method continuously detects the distinct repetitive events from a continuously supplied signal, the signal being supplied either in single data points or in streams of multiple data points, possibly of different numbers (data points may be derived from an analog signal). In the present invention this can be accomplished by basing candidate classification on candidate features and internal states at any point in time, which are generated from past and present information and thus do not depend on future signal components, advantageously resulting in online functionality. Of course, this requires that all other possible process steps, such as filtering or other preprocessing steps, do not use non-causal elements and can work with single data points of the continuous signal. However, in some embodiments future signal components may also be used for candidate classification on candidate features and internal states, e.g., by applying predictive methods (analytical, statistical or model-based) to further analyze the past and present information of the signal.

According to some embodiments of the present invention, the candidate features are determined from at least one of the amplitude, time, type, and validity of the previous and current candidates. Preferably, the candidate features are determined from all of the amplitude, time, type, and validity of the previous and current candidates.

As used herein, the term "amplitude" and "time" refer to the two values which are describing the candidate signal point, alternatively one could call this the "x" and "y" component of the candidates or the "value" and "timing" of the candidates or the like.

As used herein, the term "type" refers to the event type of the candidate. For example, for respiratory peaks and troughs, the type would refer to either a peak or a trough. It is also possible for a candidate to have multiple types or for the type to have multiple events as its value.

As used herein, the term "validity" refers to whether the candidate is a true event or not. In the case that the validity of the candidate has not yet been determined by the candidate classification, the value can also be undefined or a default value. In particular, validity may be represented by an internal state of the method according to the present invention as a Boolean variable.

Advantageously, the candidate features that are determined from at least one or all of the amplitude, time, type, and validity of the previous and current candidates are thus not based on signal components outside the detected candidates, which means that these signal components or parts thereof can be erased from memory, which can also be referred to as resampling, downsampling or the like, which is advantageous for devices with limited memory.

Advantageously, the candidate features that are determined from at least one or all of the amplitude, time, type and validity of the previous and current candidates may not be determined from all previous candidates, but rather from the last few candidates, which offers the possibility to remove the other previous candidates further in the past from memory, which is advantageous for devices with limited memory.

Advantageously, at least one or all of the amplitude, time, type, and validity of a candidate are part of the candidate, so that feature generation is possible from the candidates rather than from the signal, and the signal or parts of it can be removed from memory.

According to some embodiments of the present invention, portions of the signal are erased from memory after candidate detection, while the candidates are retained, which is advantageous for devices with limited memory.

Advantageously, the beneficial memory reduction can be achieved in the present invention by several means, all of which arise from the advantageous structure of the present method in this respect, and some of which have been previously explained.

According to some embodiments of the present invention, the candidate features comprise at least one or all of the amplitude, peak-to-trough amplitude, and time distance to previous candidates, which can all be generated from at least one or all of the amplitude, time, type, and validity of the previous and current candidates and thus again are not based on signal components outside the detected candidates which means that these signal components or parts thereof can be erased from memory, which is advantageous for devices with limited memory. Specifically, the candidate features amplitude, peak-to-trough amplitude and time distance to previous candidates have a limited amount of complexity and thus in most implementations a small requirement to computational resources but a high content of information and discriminatory power as to whether or not the candidates are true events. The amount of complexity of signal and thus the requirement on memory and computational resources may thus be limited by at least partly reducing the signal to candidate features.

As used herein, the term "current candidate" refers to the candidate that is at this stage of the method in processing and coherently the term "previous candidates" refers to the candidates that were at an earlier stage of the method in processing. The entire method, and thus the generation of candidate features, operates in temporal correspondence with the time signal, so that candidates that were in processing at a previous time are also candidates that have a previous time component. Advantageously, the entire method, and thus the feature generation, operates in temporal correspondence with the time signal, so that candidates that were in processing at an earlier stage of the method are also candidates that have an earlier time point in the signal.

As used herein, the term "peak-to-trough amplitude" refers to the amplitude between a peak and a trough, where the current candidate is defined as one of the two and the other is at an earlier time in the signal.

As used herein, the term "time distance" refers to the distance with regards to the time component of the candidates, which is interchangeable with the expression "temporal distance", "sample distance", or "x distance" or the like. Thus, the time distance to previous candidates refers to the amount of time, samples, or the like to one of the previous candidates. This could be the time distance of the current candidate to the last candidate or the second last candidate or to the last valid candidate or to the last candidate of the same type or to the last candidate of another type or to the last valid candidate of the same or another type, or the like or any combination thereof. All of these can be generated from at least one or all of the time, type, and validity of the previous and current candidates.

According to some embodiments of the present invention, the at least one internal state is physiologically interpretable, which is advantageous in terms of comprehensibility and often complexity, and therefore requires a small amount of computing resources in most implementations. With respect to internal states and candidate features, a strong correlation between good comprehensibility and low complexity is often present, such that physiologically interpretable internal states have a low degree of complexity in most cases.

According to some embodiments of the present invention, the at least one internal state comprises noise level and/or signal level, both of which are specifically favorable as they have a limited amount of complexity and thus in most implementations a small requirement to computational resources but a high content of information and discriminatory power as to whether or not the candidates are true events.

According to some embodiments of the present invention, the method is applied recursively to a portion of the signal, advantageously to a portion of the signal in which no event or not all events have been detected for some time. This can be particularly useful for highly variable signals where the amplitude or other characteristics change drastically and therefore the detection does not work adequately. For example, with respiratory signals, the amplitude of the signal can change drastically and rapidly due to guided breathing or physical activity.

Certain embodiments of the present disclosure may include some, all, or none of the above advantages. One or more technical advantages may be readily apparent to those skilled in the art from the figures, descriptions, and claims included herein. Moreover, while specific advantages have been enumerated above, various embodiments may include all, some, or none of the enumerated advantages. In addition to the exemplary aspects and embodiments described above, further aspects and embodiments will become apparent by reference to the figures and by the study of the following detailed descriptions.

Another aspect of the present invention relates to a device for data processing, comprising a signal input for receiving a signal; a memory; and a processor, configured for carrying out the steps of a method according to the present invention.

As used herein, the term "signal input" refers to an interface which allows the device to receive a signal, especially a biomedical signal. The interface can be a structural element (e.g., an electrical port or connection socket) or a virtual instance (e.g., an application programming interface, API, for connecting the device to a signal source component).

As used herein, the term "memory" refers to any type of volatile or non-volatile storage for storing information. In particular, the memory may be a virtual or cloud-based memory. It may be used to at least temporarily store the signal (or at least parts of the signal) and a representation of at least one internal state, a generated feature and/or a candidate classification. The memory may store an implementation of a method according to the present invention for being executed on a processor.

As used herein, the term "processor" particularly refers to an electric microprocessor which may be configured for carrying out the steps of a method according to the present invention. A codified representation of the steps of said method may be stored in the memory as machine or program code to be executed by the processor. However, a representation of the steps of said method may also be temporarily or permanently implemented in the processor, e.g., the processor may be a programmed FPGA or the like.

Another aspect of the present invention discloses a system for detecting at least two distinct repetitive events, especially peaks and troughs, from a signal, comprising a device for data processing according to the present invention. Beside said device, the system may further comprise at least one sensors for acquiring a signal, especially a biological signal, and a battery for energy supply. The system may further comprise means for communicating with external devices, in particular means for wireless communication.

All embodiments described in this specification may be advantageously combined with one another to the extent that their respective features are compatible. In particular, the expressions "according to some embodiments", "in an embodiment", "an embodiment of the invention provides" etc. mean that the respective features may or may not be part of specific embodiments of the present invention.

**Brief Description of the Drawings**

Examples illustrative of embodiments are described below with reference to the figures attached hereto. In the figures, identical structures, elements, or parts that appear in more than one figure are generally labeled with the same numeral in all the figures in which they appear. Alternatively, elements or parts that appear in more than one figure may be labeled with different numerals in the different figures in which they appear. Dimensions of components and features shown in the figures are generally chosen for convenience and clarity of presentation and are not necessarily shown in scale. The figures are listed below.

Fig. 1    schematically illustrates a flow chart of a method according to the present invention;

Fig. 2    schematically illustrates a portion of an exemplarily signal with some candidates according to some embodiments of the present invention;

Fig. 3    schematically illustrates a portion of an exemplarily signal with some candidate features according to some embodiments of the present invention; and

Fig. 4    schematically illustrates an exemplary embodiment of a device for data processing according to the present invention.

**Detailed Description of the Drawings**

In the following description, various aspects of the disclosure will be described. For the purpose of explanation, specific configurations and details are set forth in order to provide a thorough understanding of the different aspects of the disclosure. However, it will also be apparent to one skilled in the art that the disclosure may be practiced without specific details being presented herein. Furthermore, well-known features may be omitted or simplified in order not to obscure the disclosure.

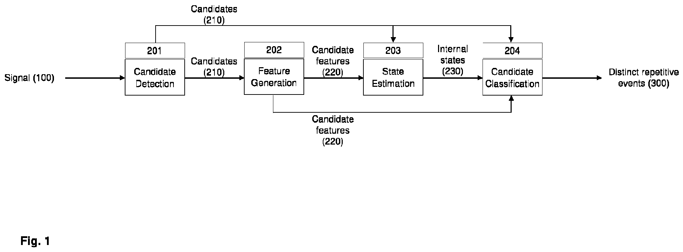

Reference is now made to Fig. 1, which schematically illustrates a flow chart of a method according to the present invention. The steps of the method are shown as candidate detection 201, feature generation 202, state estimation 203 and candidate classification 204.

A signal 100, such as a respiration signal, is provided to the method, and respiratory peaks and troughs are to be detected and returned as distinct repetitive events 300.

Before the step of candidate detection 201, some specific pre-processing steps can be performed on the signal, for example, bandpass filtering, which is often a useful addition, but is not necessary for the method to work. However, it should be noted that preferably only causal filtering should be used, otherwise the online functionality of the method may be lost.

During the candidate detection 201, candidates 210 for both respiratory peaks and troughs are detected, e.g., by detecting local extrema or by determining maxima and minima after zero crossings. The exact implementation of candidate detection 201 is interchangeable, as the present invention focuses on the evaluation of the detected candidates 210, and equivalent results can be obtained with many different candidate detections 201.

After the candidate detection 201, according to some embodiments, it is possible to erase portions of the signal 100 from memory, while the candidates 210 are retained. There might be various reasons though not to keep only the data points of the candidates 210, for example, it makes sense to keep a certain amount of signal in memory because of the implementation of online filters, but this amount is relatively small, depending on filter type and parameters. Similarly, the start and end points of breaths and thus between respiratory peaks and troughs and thus definitely outside the data points of the candidates 210 may be of interest. However, depending on the application and implementation, a large reduction in the amount of required memory is possible.

Subsequently, candidate features 220 are detected within the step of feature generation 202. According to some embodiments, the candidate features 220 are determined from at least one or all of the amplitude, time, type and validity of the previous and current candidates 210. According to some embodiments, the features could comprise at least one or all of the amplitude of the current candidate, the peak-to-valley amplitude, generated from the amplitude of the current candidate and the amplitude of the previous candidate with a different candidate type than the current candidate, and the time distance to the previous candidate, generated from the time of the current candidate and the time of the last previous candidate or the last previous candidate with a different candidate type than the current candidate.

Further, the step of state estimation 203 follows, in which the internal states 230 are adjusted based on the candidate features 220. The internal states 220 may be adjusted in a variety of ways. According to some embodiments, the internal states 220 comprise the noise level and the signal level that attempt to estimate the amplitude level of the noise of the provided signal 100 and the amplitude level of at least one of the distinct repetitive events 300, such as the respiratory peaks. It is also possible to generate a threshold level from these signal and noise levels as a further internal state 220. The internal states 220 may be exemplarily adjusted using a leaky update mechanism that, depending on the validity of the previous candidates, either leakily decreases its state or adjusts its value in a weighted manner.

At last, in the step of candidate classification 204, the distinct repetitive events 300 are determined and returned from the candidates 210 based on the candidate features 220 and the internal states 230. For this purpose, it might be useful to compare some candidate features 220 with some internal states 230 and decide according to this relationship whether the respective candidate 210 is a distinct repetitive event 300 or not.

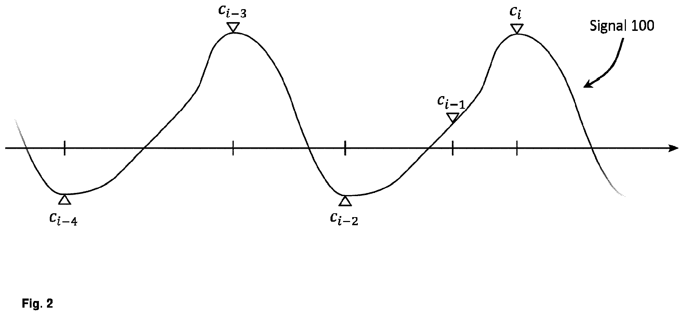

Reference is now made to Fig. 2, which schematically illustrates a portion of a signal 100 with some candidates 210 according to some embodiments of the present invention. As an example, the current candidate in process in the current phase of the procedure is called candidate $c_i$. Consequently, the previous candidates are labeled $c_{i-1}$, $c_{i-2}$, $c_{i-3}$, and $c_{i-4}$. For each candidate, there is time, amplitude, type, and validity, where validity is undetermined for $c_i$ and invalid for $c_{i-1}$ and valid for $c_{i-2}$, $c_{i-3}$, and $c_{i-4}$, and type being peak for $c_i$, $c_{i-1}$, and $c_{i-3}$ and trough for $c_{i-2}$ and $c_{i-4}$.

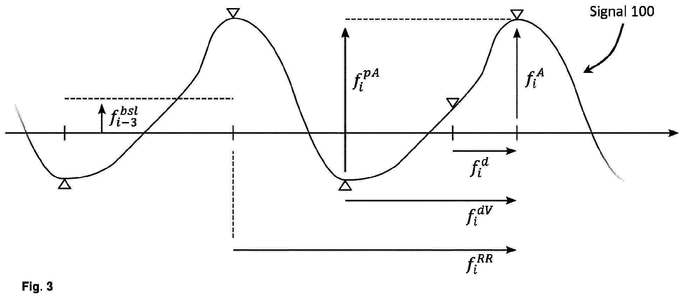

Reference is now made to Fig. 3, which schematically illustrates a portion of a signal 100 with some candidate features 220 according to some embodiments of the present invention. As an example, the generated features comprise amplitude and temporal features. The amplitude features comprise for the current candidate $c_i$ the candidate's amplitude $f_i^A$ and peak to trough amplitude $f_i^{pA}$, where the current candidate $c_i$ is the peak and the last valid candidate of the opposite type is $c_{i-2}$. The temporal features comprise the temporal distance to the last candidate $f_i^d$ and the temporal distance to the last valid candidate $f_i^{dV}$ and the temporal distance to the last valid candidate of the same type as the current candidate $f_i^{RR}$. Additionally there is the mean of the amplitudes between two opposite types as local baseline feature $f_{i-3}^{bsl}$, in this example for the candidate $c_{i-3}$.

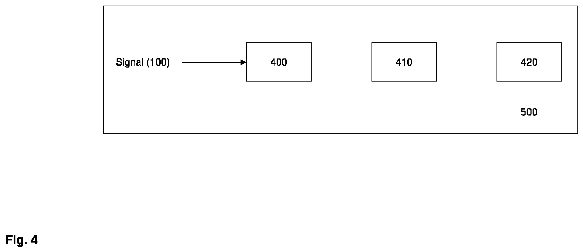

Reference is now made to Fig. 4, which schematically illustrates an exemplary embodiment of a device 500 for data processing according to the present invention. The device 500 comprises a signal input 400 for receiving a signal 100; a memory 410; and a processor 420, configured for carrying out the steps of the method according to the present invention.

The terminology used herein is for the purpose of describing particular embodiments only and is not intended to be limiting. As used herein, the singular forms "a", "an" and "the" are intended to include the plural forms as well, unless the context clearly indicates otherwise. It will be further understood that the terms "comprises" or "comprising," when used in this specification, specify the presence of stated features, integers, steps, operations, elements, or components, but do not preclude or rule out the presence or addition of one or more other features, integers, steps, operations, elements, components, or groups thereof.

While a number of exemplary aspects and embodiments have been discussed above, those of skill in the art will recognize certain modifications, additions, and sub-combinations thereof. It is therefore intended that the following appended claims and claims hereafter introduced be interpreted to include all such modifications, additions, and sub-combinations as are within their scope.

## Reference Signs

| | |
|---|---|
| 100 | signal |
| 201 | candidate detection |
| 202 | feature generation |
| 203 | state estimation |
| 204 | candidate classification |
| 210 | candidates |
| 220 | candidate features |
| 230 | internal state |
| 300 | distinct repetitive events |
| 400 | signal input |
| 410 | memory |
| 420 | processor |
| 500 | device |

## Claims

1. A method for detecting at least two distinct repetitive events (300), especially peaks and troughs, from a signal (100), the method comprising the steps of:

   a candidate detection (201) that selects respective candidates (210) for the distinct repetitive events (300) from the signal (100);
   a feature generation (202) in which candidate features (240) are captured;
   a state estimation (203) that adjusts at least one internal state (230) based on at least one candidate feature (220);
   a candidate classification (204) that determines the distinct repetitive events (300) from the respective candidates (210) based on at least one candidate feature (220) and at least one internal state (230).

2. Method according to claim 1, wherein the signal (100) is a biomedical signal.

3. Method according to any one of the preceding claims, wherein the signal (100) is a respiratory signal.

4. Method according to any one of the preceding claims, wherein the at least two distinct repetitive events (300) occur consecutively.

5. Method according to any one of the preceding claims, wherein the method continuously detects the at least two distinct repetitive events (300) from a continuously supplied signal (100).

6. Method according to at least one of the previous claims, wherein the candidate features (220) are determined from at least of the amplitude, time, type, and validity of the previous and current candidates (210).

7. Method according to any one of the preceding claims, wherein portions of the signal (100) are erased from memory after candidate detection (201), while the candidates (210) are retained.

8. Method according to any one of the preceding claims, wherein the candidate features (220) comprise at least one of the amplitude, peak-to-trough amplitude, and time distance to previous candidates (210).

9. Method according to any one of the preceding claims, wherein the at least one internal state (230) is physiologically interpretable.

10. Method according to any one of the preceding claims, wherein the at least one internal state (230) comprises noise level and/or signal level.

11. Method according to any one of the preceding claims, wherein the method is applied recursively to a portion of the signal (100).

12. Device (500) for data processing, comprising:

   a signal input (400) for receiving a signal (100);
   a memory (410); and
   a processor (420), configured for carrying out the steps of the method according to any one of the preceding claims.

13. System for detecting at least two distinct repetitive events (300), especially peaks and troughs, from a signal (100), comprising a device (500) for data processing comprising according to claim 12.

Fig. 1

Fig. 2

Fig. 3

Signal (100) ———→ | 400 |    | 410 |    | 420 |

500

**Fig. 4**

Europäisches
Patentamt

European
Patent Office

Office européen
des brevets

# EUROPEAN SEARCH REPORT

**Application Number**

EP 23 16 1315

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DALUWATTE CHATHURI ET AL: "A robust detection algorithm to identify breathing peaks in respiration signals from spontaneously breathing subjects", COMPUTING IN CARDIOLOGY, N/A, 6 September 2015 (2015-09-06), pages 297-300, XP032865145, ISSN: 2325-8861, DOI: 10.1109/CIC.2015.7408645 ISBN: 978-1-4799-0884-4 [retrieved on 2016-02-16] * the whole document * | 1-13 | INV. A61B5/08 A61B5/00 |
| X | US 2012/253216 A1 (FU YONGJI [US] ET AL) 4 October 2012 (2012-10-04) * paragraphs [0042] - [0047]; figures 9-13 * | 1-6,8, 12,13 | |
| X | CN 114 938 951 A (NANJING TUODAO MEDICAL TECH CO LTD) 26 August 2022 (2022-08-26) * the whole document * | 1-3,7,11 | |
| A | CHAN ALEXANDER M ET AL: "Ambulatory respiratory rate detection using ECG and a triaxial accelerometer", ENGINEERING IN MEDICINE AND BIOLOGY SOCIETY (EMBC), 2013 34TH ANNUAL INTERNATIONAL CONFERENCE OF THE IEEE, IEEE, 3 July 2013 (2013-07-03), pages 4058-4061, XP032489033, ISSN: 1557-170X, DOI: 10.1109/EMBC.2013.6610436 [retrieved on 2013-09-25] * Section D. Peak-picking and respiratory rate estimation * | 1 | |

**TECHNICAL FIELDS SEARCHED (IPC)**

A61B

-/--

The present search report has been drawn up for all claims

| Place of search | Date of completion of the search | Examiner |
|---|---|---|
| Berlin | 30 June 2023 | Kronberger, Raphael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 23 16 1315

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2019/029626 A1 (FENG TAO [US] ET AL) 31 January 2019 (2019-01-31) * the whole document * ----- | 1 | |
| A | WO 00/21438 A1 (UNIV FLORIDA [US]) 20 April 2000 (2000-04-20) * figures 9-15 * ----- | 1 | |
| A | US 2013/345585 A1 (GOPAL SAMY MATHAN KUMAR [DE] ET AL) 26 December 2013 (2013-12-26) * paragraphs [0040] - [0042]; figure 4 * ----- | 1 | |

TECHNICAL FIELDS
SEARCHED        (IPC)

The present search report has been drawn up for all claims

| Place of search | Date of completion of the search | Examiner |
|---|---|---|
| Berlin | 30 June 2023 | Kronberger, Raphael |

EPO FORM 1503 03.82 (P04C01)

## ANNEX TO THE EUROPEAN SEARCH REPORT
## ON EUROPEAN PATENT APPLICATION NO.

EP 23 16 1315

This annex lists the patent family members relating to the patent documents cited in the above-mentioned European search report.
The members are as contained in the European Patent Office EDP file on
The European Patent Office is in no way liable for these particulars which are merely given for the purpose of information.

30-06-2023

| Patent document cited in search report | | Publication date | Patent family member(s) | | Publication date |
|---|---|---|---|---|---|
| US 2012253216 | A1 | 04-10-2012 | US 2012253216 A1 | | 04-10-2012 |
| | | | WO 2012133930 A1 | | 04-10-2012 |
| CN 114938951 | A | 26-08-2022 | NONE | | |
| US 2019029626 | A1 | 31-01-2019 | CN 109077745 A | | 25-12-2018 |
| | | | US 2019029626 A1 | | 31-01-2019 |
| WO 0021438 | A1 | 20-04-2000 | AU 1118500 A | | 01-05-2000 |
| | | | WO 0021438 A1 | | 20-04-2000 |
| US 2013345585 | A1 | 26-12-2013 | BR 112013022900 A2 | | 14-11-2017 |
| | | | CN 103429150 A | | 04-12-2013 |
| | | | EP 2683296 A1 | | 15-01-2014 |
| | | | JP 6129082 B2 | | 17-05-2017 |
| | | | JP 2014511250 A | | 15-05-2014 |
| | | | RU 2013145520 A | | 20-04-2015 |
| | | | US 2013345585 A1 | | 26-12-2013 |
| | | | WO 2012123828 A1 | | 20-09-2012 |

EPO FORM P0459

For more details about this annex : see Official Journal of the European Patent Office, No. 12/82